# EUROPÄISCHE PATENTANMELDUNG

(11) **EP 1 927 655 A1**
(43) Veröffentlichungstag der Anmeldung: **04.06.2008**
(21) Anmeldenummer: 07121763.2
(22) Anmeldetag: 28.11.2007
(51) Int. Cl.: C12N 1/20, B09C 1/10

(54) **Verfahren zur Selektion aus Böden isolierten Mikroorganismen**

(30) Priorität: 29.11.2006 DE 102006056341
(71) Anmelder: Flughafen München GmbH, 85326 München (DE); EMC GmbH, 99086 Erfurt (DE)
(72) Erfinder: Totsche, Kai Uwe, 95463 Bindlach (DE)
(74) Vertreter: Thun, Clemens

(57) **Zusammenfassung**

Die vorliegende Erfindung beschreibt ein Verfahren zur Selektion von Mikroorganismen aus Böden, die zum Abbau von bestimmten organischen Substanzen unter bestimmten Umgebungsbedingungen geeignet sind. Das Verfahren umfasst die Perkolation von Bodenmaterial mit einer wässrigen Lösung unter Bildung von Sickerwasser, die Inkubation des Sickerwassers und die Identifizierung der Mikroorganismen.

## Beschreibung

Die vorliegende Erfindung beschreibt ein Verfahren zur Selektion von Mikroorganismen aus Böden, die zum Abbau von bestimmten organischen Substanzen unter bestimmten Umgebungsbedingungen geeignet sind.

Seit Jahren haben die Schädigungen der Umwelt weltweit erhebliche Ausmaße angenommen und drohen die Lebensbedingungen auf der Erde nachhaltig zu verschlechtern. Dabei verursachen die Industrieländer mit ihrer hohen industriellen Produktion und Emission von Substanzen, die aus diesen Produktionsprozessen stammen die bei Weitem stärksten Schädigungen. So ist insbesondere die Kontamination mit organischen Substanzen in hohem Maße unweltschädlich. Sowohl die Luft als auch Wasser und die Böden sind betroffen. Insbesondere die Kontamination des Erdreichs, d. h. des Bodens stellt eine besondere Gefahr dar. Durch den Eintrag von umweltschädlichen Substanzen in den Boden können dessen Funktionen nachhaltig gestört werden. Es kann aber auch nachfolgend zu einer schwerwiegenden Verschmutzung des Grundwassers kommen.

Insbesondere aus ökologischen Erwägungen ist ein biologischer Abbau der belastenden, organischen Stoffe im Boden selbstverständlich wünschenswert.

Es ist daher Aufgabe der vorliegenden Erfindung, eine Technologie zu entwickeln, mit der diesen Gefahren begegnet werden kann, indem Mikroorganismen zur Verfügung gestellt werden, die organische Schadstoffe auf natürliche, biologische Weise in Böden, aber auch in wässrigen Medien (Grundwasser, Abwasser, Niederschlag etc.) abzubauen vermögen.

Mit dem erfindungsgemäßen Verfahren zur Selektion von Mikroorganismen aus Böden, die zum Abbau von bestimmten organischen Substanzen unter bestimmten Umgebungsbedingungen geeignet sind, ist diese Aufgabe gelöst worden.

Das erfindungsgemäße Verfahren weist die folgenden Schritte auf:
- Perkolation von Bodenmaterial mit einer wässrigen Lösung unter Bildung von Sickerwasser,
- Inkubation des Sickerwassers und
- Identifizierung der Mikroorganismen.

Die Unteransprüche definieren bevorzugte Ausführungsformen des erfindungsgemäßen Verfahrens.

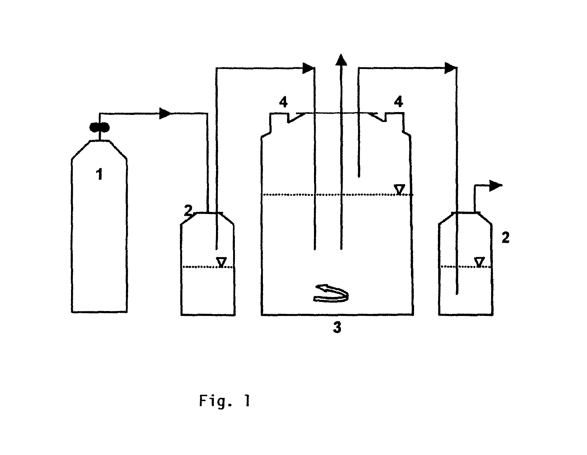

Figur 1 zeigt den schematischen Aufbau einer Versuchsanordnung, die beispielsweise in einem Inkubationsversuch des Sickerwassers gemäß dem erfindungsgemäßen Verfahren verwendet werden kann.

Natürliche Böden enthalten eine Vielzahl von Mikroorganismen, die ein breites Spektrum von organischen Substanzen unter verschiedensten chemischen und physikalischen Bedingungen abbauen können. Mit "organischen Substanzen" sind solche Stoffe gemeint, die zu der Klasse der organischen Stoffe beziehungsweise organischen Stoffmischungen gehören.

Für sehr viele Aufgabenstellungen können daher mit dem erfindungsgemäßen Verfahren Mikroorganismen aus Böden selektiert, isoliert und identifiziert werden, die dann die Basis von Mittel und Verfahren darstellen, mit denen die jeweils vorliegenden organischen Substanzen unter den jeweils vorliegenden Bedingungen biologisch abgebaut werden können. Die Selektion solcher robuster, toleranter Mikroorganismen vergrößert das Spektrum an Einsatzbedingungen, unter denen die auf der Basis der selektierten Mikroorganismen erstellten Verfahren und Mittel erforderlich angewendet beziehungsweise eingesetzt werden können.

Als Ausgangsmaterial sind vor allen Dingen solche Böden geeignet, die in der Vergangenheit bereits den betreffenden organischen Substanzen ausgesetzt waren, d. h. es wird bevorzugt Bodenmaterial verwendet, das mit den organischen Substanzen kontaminiert wurde oder kontaminiert wird.

Im ersten Verfahrensschritt wird das Bodenmaterial mit einer wässrigen Lösung perkoliert, die vorzugsweise die organischen Substanzen enthält, die durch die Mikroorganismen abgebaut werden. Dadurch erhält man ein Sickerwasser, das ein Konsortium von bodenbürtigen Mikroorganismen enthält. Das Sickerwasser wird von Spezies dominiert, die die organische Substanz als Nahrungsquelle nutzen können, also zu einem Abbau der organischen Substanz befähigt sind. Die Perkolation erfolgt unter Bedingungen, die an den späteren Einsatzzweck angepasst sind, z. B. niedrige Temperaturen. In einer bevorzugten Ausführungsform der Erfindung wird die Perkolation intermittierend durchgeführt.

Die wässrige Perkolationslösung kann neben den organischen Substanzen in Abhängigkeit von deren Eigenschaften und dem späteren Verwendungszweck der zu selektierenden Mikroorganismen noch weitere Additive enthalten. Als Additive können beispielsweise Nährstoffe, Co-Substrate, Wuchsstoffe, Vitamine, Mobilitätsvermittler, Substanzen zur Kontrolle der Konzentration der organischen Substanzen, Spurenelemente, Elektronenakzeptoren, in solchen Mengen zugegeben werden, die einen vollständigen Abbau ermöglichen und einen bestimmten Abbauweg befördern.

Diese Additive dienen weiterhin dazu, bestimmte Interaktionen zwischen dem Sickerwasser und den abiotischen Bestandteilen des Bodens zu fördern beziehungsweise zu unterdrücken und/oder um bestimmte chemische Lebensbedingungen im Sickerwasser für die Mikroorganismen einzustellen.

Zielstellung für die Perkolation des Bodenmaterials ist es, dass sich im Sickerwasser möglichst ausschließlich solche Mikroorganismen anreichern, die zum Abbau der organischen Substanzen unter den gegebenen Bedingungen befähigt sind. Dieses Ziel wird durch eine intermittierende Perkolation des Bodenmaterials erreicht. In den Ruhephasen zwischen zwei Perkolationsphasen findet eine In-Situ-Inkubation des Sickerwassers im Bodenmaterial statt. Während dieser In-Situ-Inkubation werden die Mikroorganismen selektiert und angereichert, die zum Abbau der organischen Substanz befähigt sind. Bei der anschließenden Perkolationsphase werden diese selektierten und angereicherten Mikroorganismen mit dem Sickerwasser ausgewaschen.

Die Perkolation wird maßgeblich durch den Wassergehalt des Bodens, die Perkolationsrate, Zeitpunkt und Dauer von Fließunterbrechungen sowie durch eine optionale Sickerwasserkreislaufführung bestimmt. Der volumetrische Wassergehalt kann dabei je nach Milieubedingungen zwischen vollständiger Sättigung und nahezu trockenen Bedingungen eingestellt werden. Die Dauer der Flussunterbrechungen dienen der In-Situ-Inkubation und können je nach Abbaubedingungen von wenigen Stunden bis hin zu Wochen und Monaten dauern. Die Flussunterbrechungen starten dabei frühestens nach Einstellung des gewünschten Wassergehaltes, können aber auch nach einer längeren Perkolationszeit ausgelöst werden.

Die Perkolation des Bodens zur Gewinnung des Sickerwassers kann sowohl im Freiland als auch im Labor durchgeführt werden. Bei der Sickerwassergewinnung im Freiland werden bevorzugt Lysimeter, Saugkerzen, Saugplatten oder Drainagen verwendet. Bei der Durchführung der Perkolation im Labor hat sich eine Durchführung in Lysimetern oder Bodensäulen als praktikabel erwiesen.

Die Temperatur während der Perkolation ist in Abhängigkeit der zu isolierenden Mikroorganismen und der späteren Verwendung derselben festzulegen. Normalerweise wird die Perkolation bei einer Temperatur im Bereich von 0 °C bis 40 °C durchgeführt.

Es hat sich herausgestellt, dass das Sickerwasser bevorzugt im Kreislauf geführt wird.

Nach der Perkolation wird das Sickerwasser fraktioniert, in geeigneten Behältern gesammelt und für die Inkubationsversuche nach üblichen Verfahren homogenisiert.

Nach der Gewinnung des Sickerwassers werden mit diesem Inkubationsversuche unter bestimmten chemischen und physikalischen Umweltbedingungen durchgeführt. Dies umfasst die Einstellung eines geeigneten pH-Wertes, einer geeigneten Temperatur, einer geeigneten Ionenstärke sowie einer geeigneten chemischen Zusammensetzung der Lösung. Dadurch werden die Spezies selektiert, die die organische Substanz unter den gegebenen Bedingungen am besten abbauen können.

Die Inkubation kann in wässriger Phase erfolgen, oder sie kann auch in einem System aus wässriger Phase und Festphase durchgeführt werden.

Bei den Inkubationsversuchen sind die einzustellenden chemischen und physikalischen Umweltbedingungen aufgabenspezifisch festzulegen. Insbesondere ist hier maßgeblich, unter welchen Bedingungen die Mikroorganismen später zur Anwendung gelangen sollen.

Im Gegensatz zur Perkolation verfügen die Mikroorganismen nicht mehr über ihren natürlichen Lebensraum, den Boden. In Abhängigkeit des Einsatzgebietes der Mikroorganismen besteht der künstliche Lebensraum bei den Inkubationsversuchen nur aus einer wässrigen Phase oder aus einer Kombination aus wässriger und (künstlicher) Festphase, zum Beispiel ein Festbettreaktor.

Während der Inkubation findet ein starkes Wachstum der Mikroorganismen statt. Während dieses Wachstumsprozesses werden die Spezies verdrängt, die die organische Substanz im künstlichen Lebensraum unter den gegebenen chemischen und physikalischen Bedingungen nicht oder nur schlecht als Nahrungsquelle nutzen können. Man erhält auf diese Weise ein Konsortium an Mikroorganismen, das die organische Substanz unter den gegebenen Bedingungen am besten abzubauen vermag.

Zur Einstellung dieser chemischen und physikalischen Umweltbedingungen gehört beispielsweise dazu, dass die Inkubation des Sickerwassers in Gegenwart der organischen Substanzen durchgeführt wird. Vorzugsweise werden ein Elektronenakzeptor, Wachstumsnährstoffe und/oder Suppine hinzugefügt.

Ein bevorzugter Elektronenakzeptor ist beispielsweise eine Nitratverbindung. Weitere mögliche Elektronenakzeptoren sind Sulfate, Eisen(III)-oxihydroxide oder Mangan(IV)-oxide. Auch H₂-Gas ist ein möglicher Elektronenakzeptor.

Zu den Wachstumsnährstoffen zählen beispielsweise Quellen für elementare Nährstoffe, wie Stickstoff, Phosphor, Kalium und Schwefel. Des Weiteren können Suppine zugeführt werden, zu denen beispielsweise Vitamine und Spurenelemente zählen. Die Vitamine werden entweder in Reinform oder in Form von Trockenhefe, Hefeextrakt oder Bierhefe verwendet.

Während der Inkubation werden physiko-chemische Parameter gemessen. Beispiele für diese physiko-chemischen Parameter sind der pH-Wert, die elektrische Leitfähigkeit, der DOC-Wert, die Trübung, der Sauerstoffgehalt, der Kohlendioxidgehalt, der Gehalt an organischen Substanzen und deren Metabolite sowie Anionen und Kationen. Die Inkubationsversuche werden durchgeführt, bis kein weiteres Wachstum der Mikroorganismen mehr zu beobachten ist.

Anschließend erfolgt die Identifizierung der selektierten Mikroorganismen. Die Identifikation der Mikroorganismen erfolgt beispielsweise mit klassischen Verfahren, wobei die ribosomale RNS mittels Polymerase-Ketten-Reaktion amplifiziert wird und anschließend sequenziert wird und mit Gen-Datenbanken verglichen wird.

Mit dem erfindungsgemäßen Verfahren lassen sich für nahezu jede organische Substanz Mikroorganismen aus Böden isolieren, die diese Substanz abbauen können oder zumindest deren Abbau beschleunigen können. Ein Beispiel für organische Substanzen, für deren Abbau mit dem erfindungsgemäßen Verfahren geeignete Mikroorganismen gefunden wurden, sind Gefrierpunkt erniedrigende Mittel, wie Enteisungsmittel. Zu diesen Enteisungsmitteln zählen unter anderem Glykole, wie Propylenglykol, Bernsteinsäure, Acetate und Formiate, beispielsweise Kaliumformiat.

Allgemein lassen sich die nach dem erfindungsgemäßen Verfahren hergestellten Mikroorganismen in mit organischen Substanzen kontaminierte Böden oder wässrigen Medien, wie beispielsweise Grundwasser, Abwasser, Niederschlag etc., einbringen. Sie können auch auf mit organischen Substanzen kontaminierte befestigte oder unbefestigte Flächen ausgetragen werden.

Mit dem erfindungsgemäßen Verfahren können durch geeignete Einstellung der chemischen und physikalischen Umweltbedingungen während der Inkubationsversuche einzelne spezifische Mikroorganismen selektiert werden. Des Weiteren können auch durch Auswählen der entsprechenden Parameter Mikroorganismen selektiert werden.

Ein Beispiel für die Anwendung der nach dem erfindungsgemäßen Verfahren selektierten Mikroorganismen ist deren Einsatz auf befestigten und unbefestigten Flächen von Flughäfen. Dort kommt es durch die Flugzeug- und Flächenenteisung im Zuge des Winterdienstes zu diffusen Einträgen von Enteisungsmittel auf unversiegelte Bodenflächen. Da die Enteisungsmittel in der Schneeauflage und/oder auf der Bodenoberfläche nur zum geringen Teil abgebaut werden, kommt es zu einem Eintrag von Enteisungsmittel in den Boden und nachfolgend auch in das Grundwasser.

Die nach dem erfindungsgemäßen Verfahren selektierten Mirkoorganismen werden zum Abbau der Enteisungsmittel als Mittel in Form einer wässrigen Lösung, die eine Sauerstoffquelle und weitere Additive enthält, zu geeigneten Zeitpunkten in entsprechender Dosierung bei niedrigen Temperaturen auf die Bodenoberfläche beziehungsweise Schneeauflage aufgebracht. An den Orten der Austragung der Mikroorganismenlösung wird der Abbau der Enteisungsmittel in der Schneeauflage beziehungsweise an der Bodenoberfläche beschleunigt. Damit wird der Eintrag von Enteisungsmittel in den Boden und nachfolgend in das Grundwasser deutlich reduziert.

Das Mittel wird auch zum Abbau von organischen Substanzen auf beliebigen Oberflächen, Böden oder in wässrigen Medien, wie beispielsweise Grundwasser, Abwasser, Niederschlag, etc., verwendet.

Das folgende Beispiel dient zur Erläuterung des erfindungsgemäßen Verfahrens.

### Beispiel

### 1. Sammeln von Bodenmaterial

Es wurde Oberboden (Grassoden und Humusschicht) vom Standort des Flughafen München verwendet. Die Böden wurden aus Bereichen gewonnen, die seit Jahren mit diffusen Einträgen von Enteisungsmitteln, wie beispielsweise Propylenglykol und Kaliumformiat, belastet sind.

### 2. Perkolation des Bodenmaterials

Die Gewinnung des Sickerwassers erfolgte durch Perkolation des gewonnenen Oberbodenmaterials unter Verwendung eines Laborlysimeters. Die Größe des Lysimeters betrug 50 cm x 50 cm mit einer Befüllungstiefe von 15 cm. Der Boden des Lysimeters wurde mit einem Edelstahlgitter und einem Maschennetz mit einer Porenweite von 50 µm ausgelegt. Der Lysimeter wurde mit einer ca. 4 cm starken Humusschicht befüllt, in die eine TDR-Sonde zur Bestimmung des volumetrischen Wassergehalts eingebaut wurde. Die Humusschicht wurde anschließend mit einer 8 bis 10 cm mächtigen Grassode bedeckt.

Für die Generierung des Sickerwassers wurde der Lysimeter mit einer Lösung bestehend aus destilliertem Wasser und Enteisungsmittel "Typ I" (ca. 75 mg 1-1 Propylenglykol) beregnet. Hierzu wurde ein hydraulischer Beregner (emc GmbH, Erfurt) verwendet, mit dem eine Beregnungsrate von 0,5 1h⁻¹ vorgegeben wurde. Die Generierung des Sickerwassers wurde bei 10 °C durchgeführt. Die Beregnung erfolgte phasenweise, wobei zwischen den Beregnungsphasen eine in-situ Inkubation möglich war.

Das Sickerwasser wurde fraktioniert, in braunen Glasflaschen gesammelt und für die Inkubationsversuche homogenisiert.

### 3. Inkubation des Sickerwassers

Die Inkubationsversuche wurden bei 4 °C ausschließlich in wässriger Phase durchgeführt. Der Zutritt von Sauerstoff wurde dadurch unterbunden, dass der Inkubator mit Stickstoff begast wurde. Weiterhin wurden im Zustrom und im Abstrom der Inkubatoren CO₂-Fallen installiert, mit denen die Zuluft von CO₂ befreit bzw. das produzierte CO₂ in der Abluft aufgefangen wurde. Die quantitative Bestimmung erfolgt durch Rücktitration mit Salzsäure.

Die Figur 1 zeigt den schematischen Aufbau einer Versuchsanordnung, die beispielsweise in einem Inkubationsversuch des Sickerwassers verwendet werden kann. Das Bezugszeichen 1 bedeutet eine Druckflasche, das Bezugszeichen 2 CO₂-Fallen, das Bezugszeichen 3 eine Inkubationsflasche mit luftdicht abschließbaren Probenahmeports und das Bezugszeichen 4 luftdicht abschließbare Flansche zur Aufnahme von Sensoren.

In den Inkubatoren wurde 11 Sickerwasser (mit ca. 10³ lebenden Zellen pro ml) vorgelegt. Es wurde 1 gl⁻¹ Propylenglykol auf Basis von Enteisungsmittel sowie die zum Abbau des Propylenglykol erforderliche Menge an Ammoniumnitrat als Sauerstoffdonator zugegeben. Ferner wurden ein Basalmedium mit 0,62 g/l (NH₄)₂SO₄, 1,00 g/l KH₂PO₄, 2,00 g/l Na₂HPO₄ x 2 H₂O, 0,06 g/l K₂SO₄, Spurenelemente und Vitamine zugesetzt.

Während des Inkubationsversuches wurden der pH-Wert, die elektrische Leitfähigkeit, der DOC-Wert, der Sauerstoffgehalt, der Kohlendioxidgehalt, der Gehalt an Propylenglykol, die Trübung und die Metabolite von Propylenglykol, Anionen (Cl⁻, NO₃⁻, PO₄³⁻, SO₄²⁻, Formiat und Acetat) sowie Kationen (A1, Ca, Fe, K, Mg, Mn, Na) bestimmt. Die Inkubationsversuche wurden durchgeführt, bis kein weiteres Wachstum der Mikroorganismen mehr zu beobachten war.

Anschließend wurden die Mikroorganismen nach üblichen Methoden gesammelt, in einem neutralen Puffer suspendiert und für die Identifizierung im Kühlen aufbewahrt.

### 4. Identifizierung der Glykol-abbauenden Mikroorganismen

Die Identifizierung erfolgte durch PCR-Amplifikation der aus den Mikroorganismen isolierten RNA, Sequenzierung derselben und Abgleich mit Gendatenbanken. Es wurden die Arten Azidovorax sp., Azospirillum lipoferum, Rhodococcus erythropolis, Pseudomonas putida, Pseudomonas brenneri identifiziert.

## Patentansprüche

1. Verfahren zur Selektion von Mikroorganismen aus Böden, die zum Abbau von bestimmten organischen Substanzen unter bestimmten Umgebungsbedingungen geeignet sind, das die Schritte aufweist:
- Perkolation von Bodenmaterial mit einer wässrigen Lösung unter Bildung von Sickerwasser,
- Inkubation des Sickerwassers und
- Identifizierung der Mikroorganismen.

2. Verfahren nach Anspruch 1,
**dadurch gekennzeichnet,**
**dass** die Perkolation intermittierend durchgeführt wird.

3. Verfahren nach Anspruch 1 und/oder 2,
**dadurch gekennzeichnet,**
**dass** ein Bodenmaterial verwendet wird, das mit den abzubauenden organischen Substanzen kontaminiert wurde oder kontaminiert wird.

4. Verfahren nach einem der Ansprüche 1 bis 3,
**dadurch gekennzeichnet,**
**dass** eine wässrige Lösung verwendet wird, welche die organischen Substanzen enthält, die durch die Mikroorganismen abgebaut werden sollen.

5. Verfahren nach einem der Ansprüche 1 bis 4,
**dadurch gekennzeichnet,**
**dass** die wässrige Lösung weiterhin Nährstoffe, Co-Substrate, Vitamine, Wuchsstoffe, Suppine, Spurenelemente, Elektronenakzeptoren,
Mobilitätsvermittler, oder Substanzen zur Kontrolle der Konzentration der organischen Substanzen enthält.

6. Verfahren nach einem der Ansprüche 1 bis 5,
**dadurch gekennzeichnet,**
**dass** die Perkolation im Freiland unter Verwendung von einem Lysimeter, Saugkerzen, Saugplatten oder Drainagen durchgeführt wird.

7. Verfahren nach einem der Ansprüche 1 bis 6,
**dadurch gekennzeichnet,**
**dass** die Perkolation im Labor unter Verwendung von einem Lysimeter oder Bodensäulen durchgeführt wird.

8. Verfahren nach einem der Ansprüche 1 bis 7,
**dadurch gekennzeichnet,**
**dass** die Perkolation bei einer Temperatur im Bereich von 0°C bis 40°C durchgeführt wird.

9. Verfahren nach einem der Ansprüche 1 bis 8,
**dadurch gekennzeichnet,**
**dass** das Sickerwasser im Kreislauf geführt wird.

10. Verfahren nach Anspruch 9,
**dadurch gekennzeichnet,**
**dass** das Sickerwasser homogenisiert wird.

11. Verfahren nach einem der Ansprüche 1 bis 10,
**dadurch gekennzeichnet,**
**dass** die Inkubation in wässriger Phase oder in einem System aus wässriger Phase und Festphase durchgeführt wird.

12. Verfahren nach Anspruch 11,
**dadurch gekennzeichnet,**
**dass** die Inkubation des Sickerwassers in Gegenwart der abzubauenden organischen Substanzen durchgeführt wird.

13. Verfahren nach Anspruch 10,
**dadurch gekennzeichnet,**
**dass** ein Sauerstoffdonator, Wachstumsnährstoffe und/oder Suppine hinzugefügt werden.

14. Verfahren nach einem der Ansprüche 1 bis 13,
**dadurch gekennzeichnet,**
**dass** während der Inkubation physiko-chemische Parameter gemessen werden.

15. Verfahren nach Anspruch 14,
**dadurch gekennzeichnet,**
**dass** der pH-Wert, die elektrische Leitfähigkeit, der DOC-Wert, die Trübung, der Sauerstoffgehalt, der Kohlendioxidgehalt, der Gehalt an organischen Substanzen und deren Metabolite sowie Anionen und Kationen gemessen werden.

16. Verfahren nach einem der Ansprüche 1 bis 15,
**dadurch gekennzeichnet,**
**dass** die die organische Substanz abbauenden Mikroorganismen identifiziert werden.

17. Verfahren nach einem der Ansprüche 1 bis 16,
**dadurch gekennzeichnet,**
**dass** die abzubauenden organischen Substanzen Gefrierpunkt erniedrigende Mittel sind.

18. Verfahren nach Anspruch 17,
**dadurch gekennzeichnet,**
**dass** die Gefrierpunkt erniedrigenden Mittel Glykole, Acetate und/oder Formiate sind.

19. Verfahren nach einem der Ansprüche 1 bis 18,
**dadurch gekennzeichnet,**
**dass** einzelne spezifische Mikroorganismen selektiert werden.

20. Verfahren nach einem der Ansprüche 1 bis 19,
**dadurch gekennzeichnet,**
**dass** eine Mischung spezifischer Mikroorganismen selektiert wird.

21. Verwendung der nach mindestens einem der Ansprüche 1 bis 20 hergestellten Mikroorganismen zur Einbringung in mit organischen Substanzen kontaminierten Böden oder wässrigen Medien oder zur Austragung auf mit organischen Substanzen kontaminierten befestigten oder unbefestigten Flächen.

22. Mittel zum Abbau von organischen Substanzen auf Oberflächen, in Böden oder in wässrige Medien, das die nach mindestens einem der Ansprüche 1 bis 20 hergestellten Mikroorganismen enthält.
